# EUROPEAN PATENT APPLICATION

(11) **EP 4 410 774 A1**
(43) Date of publication of application: **07.08.2024**
(21) Application number: 21958513.0
(22) Date of filing: 25.11.2021
(51) Int. Cl.: C07D 207/08, A61K 31/40, A61K 31/138, A61K 31/166, A61P 31/04, A23L 33/10, A61K 8/49, A61K 8/41, A61K 8/42, A61Q 17/00

(54) **ANTIBACTERIAL COMPOSITION**

(30) Priority: 27.09.2021 KR 20210127235
(71) Applicant: Seoul National University R&DB Foundation, Seoul 08826 (KR)
(72) Inventor: KWON, Yong Tae, Seoul 03080 (KR); LEE, Yoon Jee, Seoul 03080 (KR); JI, Chang Hoon, Seoul 03080 (KR); JUNG, Chan Hoon, Seoul 03080 (KR); JUNG, Chang An, Seoul 03077 (KR); KOH, Ah Ra, Seoul 03077 (KR); KIM, Hye Yeon, Seoul 03077 (KR)
(74) Representative: Dr. Schön, Neymeyr & Partner Patentanwälte mbB
(86) International application number: PCT/KR2021/017547
(87) International publication number: WO 2023/048337

(57) **Abstract**

The present specification relates to a compound represented by chemical formula 1, a salt, a stereoisomer, a solvent or a hydrate thereof, an antibacterial composition, a composition for inhibiting bacterial infections, a composition for preventing, alleviating or treating bacterial infectious disease, and a composition for preventing, alleviating or treating inflammation caused by a bacterial infection. The composition containing, as an active ingredient, a compound represented by chemical formula 1 or a salt, a stereoisomer, a solvent or a hydrate thereof, according to one aspect of the present invention, specifically binds to p62 protein so as to activate p62, thereby increasing the activity of autophagy, which is inhibited by a bacterial infection, and thus has antibacterial activity against various bacteria, can inhibit bacterial infectivity and has the excellent effect of enabling prevention, alleviation or treatment of bacterial infectious disease and inflammation caused by an infection.

## Description

### [Technical Field]

### Cross reference to related application

The present application claims priority to Korean Patent Application No. 10-2021- 0127235, filed September 27, 2021, the entire contents of which is incorporated herein for all purposes by this reference.

### Description about National Support Research and Development

This study was made by the support of Group Research Support (R&D) of the Ministry of Science and ICT under the supervision of the National Research Foundation of Korea, and the research project name is Cell Decomposition Biology Research Center (Project identification number: 1711135259). In addition, this study was made by the support of Personal Basic Research (R&D) of the Ministry of Science and ICT under the supervision of the National Research Foundation of Korea, and the research project name is Function and Mechanism of the N-degron Decomposition Pathway (Project identification number: 1711130480).

The present specification relates to an antibacterial composition, a composition for inhibiting bacterial infectivity, a composition for preventing, alleviating or treating bacterial infectious diseases, and a composition for preventing, alleviating or treating inflammation caused by bacterial infection.

### [Background Art]

Bacteria are a type of microorganism, and most prokaryotes without a nuclear membrane belong to this group. When bacteria grow to a certain size, they divide into two, each growing independently, and if the environment is right, they reproduce very quickly. Infection refers to a condition in which microorganisms, including bacteria, that have the potential to cause disease attach to the surface of the tissue of a host, such as a human, animal or plant, or invade the interior and proliferate. Infection occurs due to the correlation between the proliferative ability of bacteria and the resistance of the host.

Antibacterial agents are substances that prevent the creation or inhibit the reproduction of microorganisms, and act on bacteria that invade the human body to prevent, suppress, or treat infection. Antibacterial agents that inhibit the growth and reproduction of bacteria are classified as bacteriostatic agents, and antibacterial agents that directly kill bacteria are classified as bactericidal agents. Most antibacterial agents are effective by acting specifically on parts of bacteria that are different from human cells. Which part of bacteria they inhibit varies depending on the type of antibacterial agent. Based on their mechanism of action, it can be divided into cell wall synthesis inhibitors, cell membrane function inhibitors, protein synthesis inhibitors, nucleic acid synthesis inhibitors, and folic acid synthesis inhibitors.

Meanwhile, among antibacterial agents, those derived from secondary metabolites produced by microorganisms are called antibiotics. These are drugs that kill microorganisms or inhibit the growth of microorganisms at very low concentrations and treat infections caused by pathogens. Antibiotics are used all over the world because it shows excellent efficacy in treating antibiotic infection symptoms. The term antibiotic was first used by Waksman in the 1940s, and initially used to distinguish between the synthetic substances sulfonamides and penicillin produced by the penicillin mold (Penicillium sp.), which were mainly used at the time. Since then, as many substances exhibiting antimicrobial activity have been discovered and synthesized, it has become difficult to unambiguously define the meaning of antibiotics. Currently, antibiotics are generally accepted to mean natural, semi-synthetic, or synthetic substances that have effective antimicrobial activity at low concentrations.

However, due to the overuse of antibiotics, the so-called super-bacteria that pathogens have developed the ability to resist themselves, their resistance has gradually become stronger, and they can resist any antibiotic first appeared in Japan in 1996. As a result, problems arose in the effective use of antibiotics. In the case of antibiotics, their action of killing bacteria induces mutations in bacteria, and in the process of forming these mutations, genes that cause antibiotic resistance are created, which can lead to the creation of drug-resistant mutant bacteria. These resistant bacteria can transfer resistance genes to other bacteria, so the spread of resistant bacteria can occur quickly. Representative examples of these super-bacteria include MRSA (Methicillin-resistant Staphylococcus aureus), and VRSA (Vancomycin-resistant Staphylococcus aureus) and VRE (Vancomycin-resistant Enterococci), which have stronger antibiotic resistance. Antibiotic-resistant bacteria are resistant to antibiotics, and their resistance gradually becomes stronger and can resist any antibiotic. Accordingly, the need to develop substances or drugs that are resistant to previously reported antibiotics is being further emphasized. In other words, interest is focused on the development of drugs with new mechanisms of action that can replace existing antibacterial agents or antibiotics. Therefore, there is a need to secure target-specific search methods and new resources for the development of new active substances.

Accordingly, while researching compounds with antibacterial activity against bacteria through a new mechanism of action, the present inventors confirmed that the compound according to the present invention, its salt, stereoisomer, solvate or hydrate has antibacterial activity through a new mechanism, and completed the the present invention.

### [Detailed Description of the Invention]

### [Technical Problem]

The inventors conducted research to develop a material with antibacterial activity through a new mechanism of action. As a result of the study, it was confirmed that the compound represented by the following Chemical Formula 1, a salt, a stereoisomer, a solvate or a hydrate thereof binds to and activates p62, thereby exhibiting an antibacterial effect.

Accordingly, in one aspect, an object of the present invention is to provide a compound represented by the following Chemical Formula 1, a salt, a stereoisomer, a solvate or a hydrate thereof: 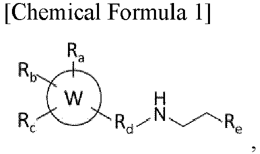 wherein in Chemical Formula 1,
W is phenyl;
Rₐ, R_{b} and R_{c} are each independently hydrogen, halogen, 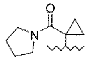 or -O-(CH₂)ₙ₁-R₁, R₁ is unsubstituted phenyl or halogen-substituted phenyl, and n1 is an integer from 1 to 3;
R_{d} is -CH₂-, -O-CH₂-CH(CH₃)-CH₂-, -O-(CH₂)ₙ₂-, -O-CH₂-CH(OH)-CH₂-, -(C=O)- or - N(CH₃)-CH₂-CH(OH)-CH₂-, and n2 is an integer of 1 to 5; and
Rₑ is -OH or -NH₂.

In another aspect, an object of the present invention is to provide a composition for antibacterial use comprising the compound represented by Chemical Formula 1, a salt, a stereoisomer, a solvate or a hydrate thereof as an active ingredient.

In another aspect, an object of the present invention is to provide a composition for inhibiting bacterial infectivity, comprising the compound represented by Chemical Formula 1, a salt, a stereoisomer, a solvate or a hydrate thereof as an active ingredient.

In another aspect, an object of the present invention is to provide a composition for preventing, alleviating or treating bacterial infectious diseases, comprising the compound represented by Chemical Formula 1, a salt, a stereoisomer, a solvate or a hydrate thereof as an active ingredient.

In another aspect, an object of the present invention is to provide a composition for preventing, alleviating or treating inflammation caused by bacterial infection, comprising the compound represented by Chemical Formula 1, a salt, a stereoisomer, a solvate or a hydrate thereof as an active ingredient.

### [Solution to Problem]

One aspect of the present invention provides a compound represented by the following Chemical Formula 1, a salt, a stereoisomer, a solvate or a hydrate thereof: 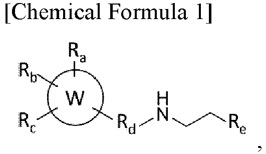 wherein in Chemical Formula 1,
W is phenyl;
Rₐ, R_{b} and R_{c} are each independently hydrogen, halogen, 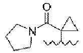 or -O-(CH₂)ₙ₁-R₁, R₁ is unsubstituted phenyl or halogen-substituted phenyl, and n1 is an integer from 1 to 3;
R_{d} is -CH₂-, -O-CH₂-CH(CH₃)-CH₂-, -O-(CH₂)ₙ₂-, -O-CH₂-CH(OH)-CH₂-, -(C=O)- or - N(CH₃)-CH₂-CH(OH)-CH₂-, and n2 is an integer of 1 to 5; and
Rₑ is -OH or -NH₂.

Another aspect of the present invention provides a composition for antibacterial use comprising the compound represented by Chemical Formula 1, a salt, a stereoisomer, a solvate or a hydrate thereof as an active ingredient.

Another aspect of the present invention provides a composition for inhibiting bacterial infectivity, comprising the compound represented by Chemical Formula 1, a salt, a stereoisomer, a solvate or a hydrate thereof as an active ingredient.

Another aspect of the present invention provides a composition for preventing, alleviating or treating bacterial infectious diseases, comprising the compound represented by Chemical Formula 1, a salt, a stereoisomer, a solvate or a hydrate thereof as an active ingredient.

Another aspect of the present invention provides a composition for preventing, alleviating or treating inflammation caused by bacterial infection, comprising the compound represented by Chemical Formula 1, a salt, a stereoisomer, a solvent or a hydrate thereof as an active ingredient.

### [Advantageous Effects of Invention]

A composition containing, as an active ingredient, a compound represented by Chemical Formula 1, a salt, a stereoisomer, a solvent or a hydrate thereof, according to one aspect of the present invention, specifically binds to p62 protein so as to activate p62, thereby increasing the activity of autophagy, which is inhibited by a bacterial infection, and thus has antibacterial activity against various bacteria, can inhibit bacterial infectivity and has the excellent effect of enabling prevention, alleviation or treatment of bacterial infectious disease and inflammation caused by an infection. Therefore, the composition can be effectively used in therapeutic agents, food, food additives, feed additives, disinfectants, cosmetics and the like.

### [Brief Description of Drawings]

FIGs. 1A to 1J show changes in p62 and LC3 expression levels, changes in expression levels of autophagy-related genes, and changes in infectivity of S. *Typhimurium* during inhibition of the expression of autophagy genes when J774A.1 macrophages and HeLa cells are infected with *Salmonella Typhimurium* according to an embodiment of the present invention.
FIGs. 2A to 2D show changes in p62, NBR1, Optinurin, NDP52, TAX1BP1 genes, and changes in the genes during inhibition of the expression of p62 when HeLa cells are infected with *S*. *Typhimurium,* including *E*. *coli,* according to an embodiment of the present invention.
FIGs. 3 and 4 show the results of colony formation analysis when a compound according to one aspect of the present invention is treated with *Salmonella Typhimurium-*infected Raw264.7 macrophages.

### [Best Mode of Carrying Out the Invention]

Hereinafter, the present invention sill be described in detail.

In one aspect, the present invention provides a compound represented by the following Chemical Formula 1, a salt, a stereoisomer, a solvate or a hydrate thereof: 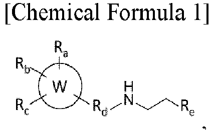 wherein in Chemical Formula 1,
W is phenyl;
Rₐ, R_{b} and R_{c} are each independently hydrogen, halogen, 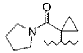 or -O-(CH₂)ₙ₁-R₁, R₁ is unsubstituted phenyl or halogen-substituted phenyl, and n1 is an integer from 1 to 3;
R_{d} is -CH₂-, -O-CH₂-CH(CH₃)-CH₂-, -O-(CH₂)ₙ₂-, -O-CH₂-CH(OH)-CH₂-, -(C=O)- or - N(CH₃)-CH₂-CH(OH)-CH₂-, and n2 is an integer of 1 to 5; and
Rₑ is -OH or -NH₂.

In one aspect of the present invention, the compound may be selected from the group consisting of compounds 1 to 11 below:
1) (1-(3-(((2-hydroxyethyl)amino)methyl)phenyl)cyclopropyl)(pyrrolidin-1-yl)methanone,
2) (1-(4-(((2-hydroxyethyl)amino)methyl)phenyl)cyclopropyl)(pyrrolidin-1-yl)methanone,
3) 2-((3-(3,4-bis((4-fluorobenzyl)oxy)phenoxy)-2-methylpropyl)amino)ethan-1-ol,
4) (*S*)-1-(3,4-bis((4-fluorobenzyl)oxy)phenoxy)-3-((2-hydroxyethyl)amino)propan-2-ol,
5) (*R*)-1-(4-((4-fluorobenzyl)oxy)phenoxy)-3-((2-hydroxyethyl)amino)propan-2-ol,
6) (*R*)-1-((2-aminoethyl)amino)-3-(3,4-bis((4-fluorobenzyl)oxy)phenoxy)propan-2-ol,
7) 3,4-bis(benzyloxy)-N-(2-hydroxyethyl)benzamide,
8) (*R*)-1-((4-((4-fluorobenzyl)oxy)phenyl)(methyl)amino)-3-((2-hydroxyethyl)amino)propan-2-ol,
9) (*S*)-1-(3-((4-fluorobenzyl)oxy)phenoxy)-3-((2-hydroxyethyl)amino)propan-2-ol,
10) (*R*)-1-(3-chloro-5-((4-fluorobenzyl)oxy)phenoxy)-3-((2-hydroxyethyl)amino)propan-2-ol, and
11) 2-((3-(3,4-bis((4-fluorobenzyl)oxy)phenoxy)propyl)amino)ethan-1-ol.

In one aspect of the invention, a "salt" may be a "pharmaceutically acceptable salt." "Pharmaceutically acceptable" means that it is capable of being approved or approved by the government or equivalent regulatory agency for use in animals, more specifically in humans, by avoiding significant toxic effects when used in typical medicinal dosages, or is listed in a pharmacopoeia or is recognized as being listed in other general pharmacopoeia.

In one aspect of the present invention, "pharmaceutically acceptable salt" means a salt according to one aspect of the present invention that is pharmaceutically acceptable and has the desired pharmacological activity of the parent compound. The salts may include (1) acid addition salt formed with inorganic acids such as hydrochloric acid, hydrobromic acid, sulfuric acid, nitric acid, phosphoric acid, etc.; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2,2,2]-oct-2-ene-1-carboxylic acid, glucoheptonic acid, 3-phenylpropionic acid, trimethylacetic acid, tert-butylacetic acid, lauryl sulfate, gluconic acid, glutamic acid, hydroxynaphthoic acid, salicylic acid, stearic acid, and muconic acid; or (2) a salt formed when an acidic proton present in the parent compound is replaced.

In one aspect of the invention, "stereoisomers" specifically include optical isomers (e.g., essentially pure enantiomers, essentially pure diastereomers, or mixtures thereof) as well as conformational isomers (i.e., isomers that differ only in the angle of one or more chemical bonds) or geometric isomers (e.g., cis-trans isomers).

In one aspect of the present invention, "essentially pure", when used in relation to, for example, enantiomers or partial isomers, means that the specific compound, for example, the enantiomer or partial isomer, present at about 90%(w/w) or more, specifically about 95%(w/w) or more, more specifically about 97%(w/w) or more or about 98%(w/w) or more, even more specifically about 99%(w/w) or more, even more and more specifically about 99.5%(w/w) or more.

In one aspect of the present invention, "hydrate" refers to a compound to which water is bound, and is a broad concept that includes embedded compounds in which there is no chemical bond between water and the compound.

In one aspect of the present invention, "solvate" refers to a higher-order compound formed between a solute molecule or ion and a solvent molecule or ion.

In another aspect, the present invention provides a composition for antibacterial use comprising the compound represented by Formula 1, a salt thereof, a stereoisomer thereof, a solvate thereof of hydrate thereof as an active ingredient.

In one aspect of the present invention, the composition may have antibacterial activity through p62 activation, or may have antibacterial activity through p62 activation and bacteriophage induction. Specifically, the composition may have antibacterial activity by binding to p62 and increasing the activity of p62, and more specifically, the composition may have antibacterial activity by binding to p62 and increasing the activity of p62, thereby inducing bacteriophages. Even more specifically, the composition may have antibacterial activity by binding to p62 and increasing the activity of p62, thereby improving the degradative flux through selective autophagy and inducing bacteriophages. According to one embodiment of the present invention, the expression of LC3 and its lipidation (LC3-II) are infected by bacterial infection, resulting in a decrease in autophagy activity (Experimental Example 1), and inhibition of p62 expression has a significant impact on the infectivity of the bacteria (Experimental Example 2). As a result, it was found that the composition according to an embodiment of the present invention is a p62 activator that binds to p62, and has an antibacterial effect by activating p62 and at the same time improving the degradative flux through selective autophagy, inhibiting bacterial infectivity, and inducing bacteriophage (Experimental Example 3).

In one aspect of the present invention, the composition may have antibacterial activity non-specifically for types of bacteria. Specifically, the composition may have antibacterial activity against Gram-negative or Gram-positive bacteria. More specifically, the gram-negative bacteria may be one or more selected from the group consisting of *Salmonella* species, *Yersinia* species, *Escherichia* species, *Chlamydia* species, *Xanthomonas* species, *Erwinia* species, *Pseudomonas* species and *Ralstonia* species. Even more specifically, the gram-negative bacteria may be one or more selected from the group consisting of *Salmonella Typhimurium, Yersinia pestis, Yersinia pseudotuberculosis, Yersinia enterocolitica, Escherichia coli, Chlamydia* species, *Xanthomonas campestris, Pseudomonas syringae* and *Ralstonia solanacearum.* Even more and more specifically, the composition may have antibacterial activity against *Salmonella Typhimurium.* Or more specifically, the gram-positive bacteria may be one or more selected from the group consisting of *Mycobacterium* species, *Streptococcus* species, *Staphylococcus* species, *Enterococcus* species and *Lactobacillus* species. Even more specifically, the composition may have antibacterial activity against one or more selected from the group consisting of *Mycobacterium tuberculosis* and *Streptococcus pyogenes.* According to one embodiment of the present invention, it was found that when the composition according to one embodiment of the present invention is treated with cells infected with *Salmonella Typhimurium,* the number of colonies produced by bacteria propagating in the cells decreases compared to the control group, thereby providing an excellent antibacterial effect (Experimental Example 3).

In one aspect of the invention, the composition may inhibit the proliferation or growth of infected bacteria by 10% or more, 15% or more, 20% or more, 25% or more, 30% or more, 35% or more, 40% or more, 45% or more. 50% or more, 55% or more, 60% or more, 65% or more, 70% or more, 75% or more, 80% or more, 85% or more, or 90% or more compared to the negative control group, but is not limited thereto.

In one aspect of the present invention, the composition may have antibacterial activity against bacteria resistant to antibiotics. The antibiotics may be one or more selected from the group consisting of Isoniazid (INH), Rifapentin (RFP), Streptomycin (SM), Ethambutol (EMB), Prothionamide (PTH), Cycloserine (CS), Ofloxacin (OFX), Pyrazinamide (PZA), Rifabutin (RBT), Moxifloxacin (MXF), and Levofloxacin (LEV). The composition has antibacterial activity by a new mechanism of action, specifically bacteriophage induction through p62 activation, and has antibacterial activity even against bacteria that have become resistant to existing antibiotics, and the types of antibiotics to which the bacteria are resistant are not limited. Specifically, in the case of antibiotic-resistant bacterial infection, the composition may inhibit the proliferation or growth of the bacteria by 10% or more, 15% or more, 20% or more, 25% or more, 30% or more, 35% or more, 40% or more, 45% or more. 50% or more, 55% or more, 60% or more, 65% or more, 70% or more, 75% or more, 80% or more, 85% or more, or 90% or more compared to the negative control group, but is not limited thereto.

In one aspect of the present invention, the compound a salt, a stereoisomer, a solvate or a hydrate thereof may be 0.001 to 1000 µM based on the total volume of the composition.

In one aspect of the present invention, the dosage of the composition may be 0.01 to 5000 mg/kg/day.

In another aspect, the present invention provides a composition for inhibiting bacterial infectivity comprising compound represented by the Chemical Formula 1, a salt, a stereoisomer, a solvate or a hydrate thereof as an active ingredient. Descriptions of the compounds, pharmaceutically acceptable salts, stereoisomers, solvates, hydrates, antibacterial activity, bacteria, antibiotic resistance, concentration, dosage, etc. are as described above.

In one aspect of the invention, "infectivity" refers to the ability of a pathogen to settle in or on the surface of the host's tissue, or the property or ability to cause infection in the host's tissue, and may mean bacterial infectivity if the pathogen is a bacterium. The bacterial infectivity can be determined by measuring the degree of colony formation (CFU analysis) in cells or individuals infected with the bacteria. The composition may inhibit bacterial infectivity by 10% or more, 15% or more, 20% or more, 25% or more, 30% or more, 35% or more, 40% or more, 45% or more. 50% or more, 55% or more, 60% or more, 65% or more, 70% or more, 75% or more, 80% or more, 85% or more, or 90% or more compared to the group before or without treatment with the composition, but is not limited thereto. Additionally, the inhibition of bacterial infectivity may be due to p62 activation.

In another aspect, the present invention provides a composition for preventing, alleviating or treating bacterial infectious diseases, comprising compound represented by the Chemical Formula 1, a salt, a stereoisomer, a solvate or a hydrate thereof as an active ingredient. Descriptions of the compounds, pharmaceutically acceptable salts, stereoisomers, solvates, hydrates, antibacterial activity, bacteria, concentration, dosage, etc. are as described above.

In one aspect of the present invention, the infectious disease may be a bacterial infectious disease. The bacterial infectious disease refers to a disease caused by bacterial infection. Specifically, the bacterial infectious disease may be one or more selected from the group consisting of pneumonia, bromatotoxism, dysentery, *Pseudomonas aeruginosa* infection, impetigo, purulent disease, acute dermatitis, cystitis, vaginitis, meningitis, typhoid, paratyphoid fever, tetanus, Lyme disease, Rocky Mountain spotted fever, cholera, leprosy, brucellosis, ehrlichiosis, Q fever, scarlet fever, listeriosis, botulism, leptospirosis, pestis, typhus, bacteremia, endocarditis, and enteritis, but is not limited thereto. In addition, the bacterial infectious disease may be a disease caused by infection with gram-negative or gram-positive bacteria, and the description of gram-negative bacteria and gram-positive bacteria is as described above. Furthermore, the bacterial infectious disease may be a disease caused by antibiotic resistance, and the description of antibiotic resistance is as described above.

In another aspect, the present invention provides a composition for preventing, alleviating or treating inflammation caused by bacterial infection, comprising compound represented by the Chemical Formula 1, a salt, a stereoisomer, a solvate or a hydrate thereof as an active ingredient. Descriptions of the compounds, pharmaceutically acceptable salts, stereoisomers, solvates, hydrates, antibacterial activity, bacteria, antibiotic resistance, concentration, dosage, etc. are as described above.

In one aspect of the present invention, the composition may reduce the expression level of pro-inflammatory cytokines or chemokines overexpressed by bacterial infection. The composition may prevent, alleviate, or treat inflammation caused by the bacterial infection.

In another aspect, the composition according to one aspect of the present invention may be a pharmaceutical, food, or cosmetic composition.

As an example, the food composition includes a health functional food composition, and the health functional food composition may be used to improve, alleviate, or prevent bacterial infectious diseases. Specifically, the bacterial infectious diseases may be one or more selected from the group consisting of pneumonia, bromatotoxism, dysentery, *Pseudomonas aeruginosa* infection, impetigo, purulent disease, acute dermatitis, cystitis, vaginitis, meningitis, typhoid, paratyphoid fever, tetanus, Lyme disease, Rocky Mountain spotted fever, cholera, leprosy, brucellosis, ehrlichiosis, Q fever, scarlet fever, listeriosis, botulism, leptospirosis, pestis, typhus, bacteremia, endocarditis, and enteritis. Alternatively, the health functional food composition may be used to improve, alleviate, or prevent inflammation caused by bacterial infection.

The health food functional composition includes various types of food additives or functional foods. It may be processed into leached tea, liquid tea, beverage, fermented milk, cheese, yogurt, juice, probiotics and health supplements containing the above composition, and may be used in the form of various other food additives.

As an example, the cosmetic composition may be provided in any formulation suitable for topical application. For example, it may be provided in the formulation of a solution, an emulsion obtained by dispersing an oil phase in an aqueous phase, an emulsion obtained by dispersing an aqueous phase in an oil phase, a suspension, solid, gel, powder, paste, foam, or aerosol composition. Compositions of these formulations may be prepared according to conventional methods in the art.

As an example, the cosmetic composition may contain, in addition to the above-mentioned substances, other ingredients that can preferably have a synergistic effect on the main effect within a range that does not impair the main effect. The cosmetic composition according to the present invention may include a substance selected from the group consisting of vitamins, high molecular weight peptides, high molecular weight polysaccharides, and sphingolipids. In addition, the cosmetic composition may include, as an example, a moisturizer, an emollient, a surfactant, an ultraviolet absorber, a preservative, a disinfectant, an antioxidant, a pH adjuster, an organic and inorganic pigment, a fragrance, a cooling agent, or an antiperspirant. The mixing amount of the above ingredients can be easily selected by a person skilled in the art within the range that does not impair the purpose and effect of the present invention, and the mixing amount may be 0.01 to 5% by weight, specifically 0.01 to 3% by weight, based on the total weight of the composition.

As an example, the pharmaceutical composition may be used to prevent, alleviate, or treat bacterial infectious diseases. Specifically, the bacterial infectious diseases may be one or more selected from the group consisting of pneumonia, bromatotoxism, dysentery, *Pseudomonas aeruginosa* infection, impetigo, purulent disease, acute dermatitis, cystitis, vaginitis, meningitis, typhoid, paratyphoid fever, tetanus, Lyme disease, Rocky Mountain spotted fever, cholera, leprosy, brucellosis, ehrlichiosis, Q fever, scarlet fever, listeriosis, botulism, leptospirosis, pestis, typhus, bacteremia, endocarditis, and enteritis. Alternatively, the pharmaceutical composition may be used to prevent, alleviate, or treat inflammation caused by bacterial infection.

Additionally, as an example, the pharmaceutical composition may be provided in any formulation suitable for topical application. For example, it may be administered orally, transdermally, intravenously, intramuscularly, or by subcutaneous injection. As an example, the pharmaceutical composition may be an injection, a solution for external use on the skin, a suspension, an emulsion, a gel, a patch, or a spray, but is not limited thereto. The formulation can be easily prepared according to conventional methods in the field, and surfactants, excipients, wetting agents, emulsification accelerators, suspending agents, salts or buffers for adjusting osmotic pressure, colorants, flavorings, stabilizers, preservatives, preservatives or other commonly used supplements may be used appropriately.

The active ingredient of the pharmaceutical composition according to an embodiment of the present invention may vary depending on the subject's age, gender, weight, pathological condition and severity, route of administration, or the judgment of the prescriber.

In another aspect, the present invention may relate to a method for inhibiting bacterial infectivity, comprising administering a composition comprising the compound represented by the Chemical Formula 1, a salt, a stereoisomer, a solvate or a hydrate thereof to a subject in need of inhibiting abacterial infectivity. In one aspect of the present invention, administration of the method may be performed according to the administration method and administration dosage described herein.

In another aspect, the present invention may relate to a method for preventing, alleviating or treating bacterial infectious diseases, comprising administering a composition comprising the compound represented by the Chemical Formula 1, a salt, a stereoisomer, a solvate or a hydrate thereof to a subject in need of preventing, alleviating or treating bacterial infectious diseases, such as pneumonia, bromatotoxism, dysentery, *Pseudomonas aeruginosa* infection, impetigo, purulent disease, acute dermatitis, cystitis, vaginitis, meningitis, typhoid, paratyphoid fever, tetanus, Lyme disease, Rocky Mountain spotted fever, cholera, leprosy, brucellosis, ehrlichiosis, Q fever, scarlet fever, listeriosis, botulism, leptospirosis, pestis, typhus, bacteremia, endocarditis, enteritis, etc.. In one aspect of the present invention, administration of the method may be performed according to the administration method and administration dosage described herein.

In another aspect, the present invention may relate to a method for preventing, alleviating or treating inflammation caused by bacterial infection, comprising administering a composition comprising the compound represented by the Chemical Formula 1, a salt, a stereoisomer, a solvate or a hydrate thereof to a subject in need of preventing, alleviating or treating inflammation caused by bacterial infection. In one aspect of the present invention, administration of the method may be performed according to the administration method and administration dosage described herein.

In another aspect, the present invention may relate to a use of the compound represented by the Chemical Formula 1, a salt, a stereoisomer, a solvate or a hydrate thereof for manufacturing an antibacterial pharmaceutical composition.

In another aspect, the present invention may relate to a use of the compound represented by the Chemical Formula 1, a salt, a stereoisomer, a solvate or a hydrate thereof for manufacturing an antibacterial food composition.

In another aspect, the present invention may relate to a use of the compound represented by the Chemical Formula 1, a salt, a stereoisomer, a solvate or a hydrate thereof for manufacturing an antibacterial cosmetic composition.

In another aspect, the present invention may relate to a use of the compound represented by the Chemical Formula 1, a salt, a stereoisomer, a solvate or a hydrate thereof for manufacturing a pharmaceutical composition for inhibiting bacterial infectivity.

In another aspect, the present invention may relate to a use of the compound represented by the Chemical Formula 1, a salt, a stereoisomer, a solvate or a hydrate thereof for manufacturing a food composition for inhibiting bacterial infectivity.

In another aspect, the present invention may relate to a use of the compound represented by the Chemical Formula 1, a salt, a stereoisomer, a solvate or a hydrate thereof for manufacturing a cosmetic composition for inhibiting bacterial infectivity.

In another aspect, the present invention may relate to a use of the compound represented by the Chemical Formula 1, a salt, a stereoisomer, a solvate or a hydrate thereof for manufacturing a pharmaceutical composition for preventing, alleviating or treating bacterial infectious diseases.

In another aspect, the present invention may relate to a use of the compound represented by the Chemical Formula 1, a salt, a stereoisomer, a solvate or a hydrate thereof for manufacturing a food composition for improving, alleviating or preventing bacterial infectious diseases.

In another aspect, the present invention may relate to a use of the compound represented by the Chemical Formula 1, a salt, a stereoisomer, a solvate or a hydrate thereof for manufacturing a cosmetic composition for improving, alleviating or preventing bacterial infectious diseases.

In another aspect, the present invention may relate to a use of the compound represented by the Chemical Formula 1, a salt, a stereoisomer, a solvate or a hydrate thereof for manufacturing a pharmaceutical composition for preventing, alleviating or treating inflammation caused by bacterial infection.

In another aspect, the present invention may relate to a use of the compound represented by the Chemical Formula 1, a salt, a stereoisomer, a solvate or a hydrate thereof for manufacturing a food composition for improving, alleviating or preventing inflammation caused by bacterial infection.

In another aspect, the present invention may relate to a use of the compound represented by the Chemical Formula 1, a salt, a stereoisomer, a solvate or a hydrate thereof for manufacturing a cosmetic composition for improving, alleviating or preventing inflammation caused by bacterial infection.

In another aspect, the present invention may relate to an antibacterial use of a composition comprising the compound represented by the Chemical Formula 1, a salt, a stereoisomer, a solvate or a hydrate thereof as an active ingredient.

In another aspect, the present invention may relate to a use of a composition comprising the compound represented by the Chemical Formula 1, a salt, a stereoisomer, a solvate or a hydrate thereof as an active ingredient, for inhibiting bacterial infectivity.

In another aspect, the present invention may relate to a use of a composition comprising the compound represented by the Chemical Formula 1, a salt, a stereoisomer, a solvate or a hydrate thereof as an active ingredient, for preventing, alleviating or treating bacterial infectious diseases.

In another aspect, the present invention may relate to a use of a composition comprising the compound represented by the Chemical Formula 1, a salt, a stereoisomer, a solvate or a hydrate thereof as an active ingredient, for preventing, alleviating or treating inflammation caused by bacterial infection.

In another aspect, the present invention may relate to a composition comprising the compound represented by the Chemical Formula 1, a salt, a stereoisomer, a solvate or a hydrate thereof as an active ingredient.

In another aspect, the present invention may relate to a composition comprising the compound represented by the Chemical Formula 1, a salt, a stereoisomer, a solvate or a hydrate thereof as an active ingredient for a use of inhibiting bacterial infectivity.

In another aspect, the present invention may relate to a composition comprising the compound represented by the Chemical Formula 1, a salt, a stereoisomer, a solvate or a hydrate thereof as an active ingredient for a use of preventing, alleviating or treating bacterial infectious diseases.

In another aspect, the present invention may relate to a composition comprising the compound represented by the Chemical Formula 1, a salt, a stereoisomer, a solvate or a hydrate thereof as an active ingredient for a use of preventing, alleviating or treating inflammation caused by bacterial infection.

Hereinafter, the configuration and effects of the present invention are described in more detail through examples, preparation examples, and experimental examples. However, these examples, preparation examples, and test examples are only provided only for illustrative purposes to aid understanding of the present invention, and the scope of the present invention is not limited thereto.

### [Preparation Example] Preparation of compounds

Examples 1 to 11, which are compounds of Chemical Formula 1 according to the present invention, were each prepared by the following methods.

**[Table 1]**

| Example No. | Chemical formula name | Structural formula |
|---|---|---|
| Example 1 | (1-(3-(((2-hydroxyethyl)amino)methyl)phenyl)cyclopropyl)(p yrrolidin-1-yl)methanone | 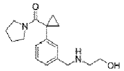 |
| Example 2 | (1-(4-(((2-hydroxyethyl)amino)methyl)phenyl)cyclopropyl)(p yrrolidin-1-yl)methanone | 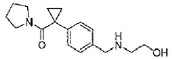 |
| Example 3 | 2-((3-(3,4-bis((4-fluorobenzyl)oxy)phenoxy)-2-methylpropyl)amino)ethan-1-ol | 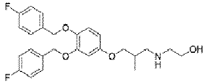 |
| Example 4 | (*S*)-1-(3,4-bis((4-fluorobenzyl)oxy)phenoxy)-3-((2-hydroxyethyl)amino)propan-2-ol | 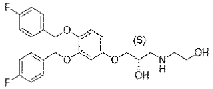 |
| Example 5 | (*R*)-1-(4-((4-fluorobenzyl)oxy)phenoxy)-3-((2-hydroxyethyl)amino)propan-2-ol | 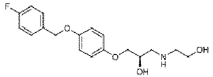 |
| Example 6 | (*R*)-1-((2-aminoethyl)amino)-3-(3,4-bis((4-fluorobenzyl)oxy)phenoxy)propan-2-ol | 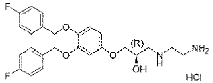 |
| Example 7 | 3,4-bis(benzyloxy)-*N*-(2-hydroxyethyl)benzamide | 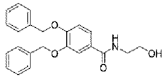 |
| Example 8 | (*R*)*-*1-((4-((4-fluorobenzyl)oxy)phenyl)(methyl)amino)-3-((2-hydroxyethyl)amino)propan-2-ol | 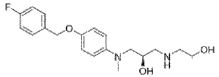 |
| Example 9 | (*S*)-1-(3-((4-fluorobenzyl)oxy)phenoxy)-3 -((2-hydroxyethyl)amino)propan-2-ol | 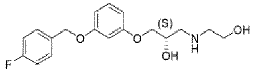 |
| Example 10 | (*R*)-1-(3-chloro-5-((4-fluorobenzyl)oxy)phenoxy)-3-((2-hydroxyethyl)amino)propan-2-ol | 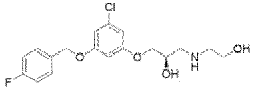 |
| Example 11 | 2-((3-(3,4-bis((4-fluorobenzyl)oxy)phenoxy)propyl)amino)ethan-1- ol | 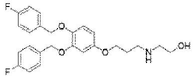 |

| | | |
|---|---|---|
| ¹H NMR spectra were recorded on Bruker Avance III 400 MHz and Bruker Fourier 300 MHz and TMS was used as an internal standard. LCMS was taken on a quadrupole Mass Spectrometer on Agilent 1260HPLC and 6120MSD (Column: C18 (50 × 4.6 mm, 5 µm) operating in ES (+) or (-) ionization mode; T = 43 °C; flow rate = 1.5 mL/min; detected wavelength: 220 nm, 254 nm). | | |

### Example 1. Preparation of (1-(3-(((2-hydroxyethyl)amino)methyl)phenyl)cyclopropyl)(pyrrolidin-1-yl)methanone

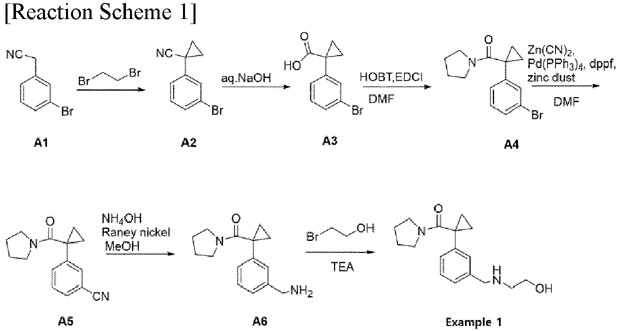

### Step 1) Synthesis of A2

A solution of **A1** (2-(3-bromophenyl)acetonitrile, 20.0 g, 103 mmol) and 1,2-dibromoethan (58.1 g, 309 mmol) in water (50.0 mL) was added benzyltriethylammonium chloride (2.34 g, 10.3 mmol). The reaction mixture was heated to 70 °C followed by dropwise addition of 50 % aqueous sodium hydroxide solution. Then the reaction was stirred at 80 °C overnight. The reaction mixture was cooled and poured into ice/water. The aqueous layer was extracted with dichloromethane. The combined organic layers were washed with brine, dried over sodium sulfate(Na₂SO₄) and concentrated. The residue was purified by silica column to give A2 (1-(3-bromophenyl)cyclopropane-1-carbonitrile, 8.00 g, yield: 36.4%) as red oil.
¹H NMR (400 MHz, CDCl₃): δ 7.41-7.43(m, 2 H), 7.22-7.24 (m, 2 H), 1.73-1.76 (m, 2 H), 1.39-1.42 (m, 2 H)

### Step 2) Synthesis of A3

A solution of **A2** (1-(3-bromophenyl)cyclopropane-1-carbonitrile, 8.00 g, 36.2 mmol) in water (50.0 mL) was added a solution of sodium hydroxide (7.24 g, 181 mmol) in water (50.0 mL). The mixture was stirred at 120 °C overnight. The reaction was cooled and poured into ice/water. The aqueous was washed with dichloromethane(DCM) and then the aqueous was acidified to pH=3 with concentrated HCI. The precipitated solid was separated by filtration and dried under reduced pressure to give the A3 (1-(3-bromophenyl)cyclopropane-1-carboxylic acid, 6.00 g, yield: 69.1%) as a white solid.

LCMS; Mass Calcd.: 241; MS Found: 242 [MS+1].

### Step 3) Synthesis of A4

A solution of A3 (1-(3-bromophenyl)cyclopropane-1-carboxylic acid, 3.00 g, 12.5 mmol), HOBT (2.54 g, 18.8 mmol), EDCI (3.61 g, 18.8 mmol) and DIEA (4.85 g, 37.6 mmol) in anhydrous dimethylformamide (DMF, 30.0 mL) was added pyrrolidine (1.60 g, 22.6 mmol) and stirred at room temperature overnight. The reaction mixture was cooled and poured into ice/water. The aqueous layer was extracted with ethyl acetate(EA). The combined organic layers were washed with brine, dried over Na₂SO₄ and concentrated. The residue was purified by silica column to give the A4 ((1-(3-bromophenyl)cyclopropyl)(pyrrolidin-1-yl)methanone, 3.00 g, yield: 83.3%.) as a yellow solid.

LCMS; Mass Calcd.: 294; MS Found: 296 [MS+2].

### Step 4) Synthesis of A5

A solution of A4 ((1-(3-bromophenyl)cyclopropyl)(pyrrolidin-1-yl)methanone, 3.00 g, 10.2 mmol), zinc cyanide (1.79 g, 15.3 mmol), Pd(PPh₃)₄ (589 mg, 0.510 mmol), dppf (565 mg, 1.02 mmol), and zinc dust (66.3 mg, 1.02 mmol) in anhydrous dimethylformamide (DMF, 30.0 mL) was stirred at 115 °C under nitrogen until all starting material was consumed (8 hrs). The reaction mixture was cooled and poured into ice/water. The aqueous layer was extracted with EA. The combined organic layers were washed with brine, dried over Na₃SO₄ and concentrated. The residue was purified by silica column to give A5 (3-(1-(pyrrolidine-1-carbonyl)cyclopropyl)benzonitrile, 2.00 g, yield: 81.6%) as yellow oil.

LCMS; Mass Calcd.: 240; MS Found: 241 [MS+1].

### Step 5) Synthesis of A6

A solution of A5 (3-(1-(pyrrolidine-1-carbonyl)cyclopropyl)benzonitrile, 2.00 g, 8.33 mmol) and ammonium hydroxide (2.00 mL) in MeOH (50.0 mL) was added Raney nickel (500 mg). The mixture was stirred under H₂ (50 psi) at room temperature overnight. The mixture was filtered through a pad of Celite and the filter cake was washed with MeOH. The combined filtrates were concentrated to give the crude A6 ((1-(3-(aminomethyl)phenyl)cyclopropyl)(pyrrolidin-1-yl)methanone, 1.50 g) as red oil and used for the next step reaction without further purification.

### Step 6) Synthesis of Example 1

A solution of A6 ((1-(3-(aminomethyl)phenyl)cyclopropyl)(pyrrolidin-1-yl)methanone, 1.50 g, 6.15 mmol) and TEA (1.24 g, 12.3 mmol) was added 2-bromoethanol (845 mg, 6.76 mmol). The reaction mixture was stirred at 50 °C overnight. The reaction mixture was cooled and poured into ice/water. The aqueous layer was extracted with EA. The combined organic layers were washed with brine, dried over Na₂SO₄ and concentrated. The residue was purified by silica column to give Example 1 ((1-(3-(((2-hydroxyethyl)amino)methyl)phenyl)cyclopropyl)(pyrrolidin-1-yl)methanone, 500 mg, yield: 28.2%) as colorless oil.

¹H NMR(400 MHz, DMSO): δ 8.25(s, 1 H), 7.17-7.29 (m, 3 H), 7.04-7.06 (m, 1 H), 3.79 (s, 2 H), 3.49-3.51 (t, 2 H), 3.31-3.34 (t, 2 H), 3.09-3.12 (t, 2 H), 2.63-2.65 (t, 2 H), 1.69-1.70 (m, 4H), 1.26-1.28 (m, 2H), 1.08-1.11 (m, 2H).

LCMS; Mass Calcd.: 288; MS Found: 289 [MS+1].

### Example 2. Preparation of (1-(4-(((2-hydroxyethyl)amino)methyl)phenyl)cyclopropyl)(pyrrolidin-1-yl)methanone

Example 2 (1-(4-(((2-hydroxyethyl)amino)methyl)phenyl)cyclopropyl)(pyrrolidin-1-yl)methanone was synthesized as colorless oil (17.0 % yield) in the same manner as in Example 1 by using 2-(4-bromophenyl)acetonitrile as a starting material instead of A1.

¹H NMR (400 MHz, DMSO): δ 8.28 (s, 1 H), 7.31-7.33 (d, 2 H), 7.11-7.13 (d, 2 H), 3.81 (s, 2 H), 3.50-3.53 (t, 2 H), 3.32 (m, 2 H), 3.11 (m, 2 H), 2.66-2.69 (t, 2 H), 1.70 (m, 4H), 1.25-1.28 (m, 2H), 1.08-1.11 (m, 2H).

LCMS; Mass Calcd.: 288; MS Found: 289 [MS+1]].

### Example 3. Preparation of 2-((3-(3,4-bis((4-fluorobenzyl)oxy)phenoxy)-2-methylpropyl)amino)ethan-1-ol

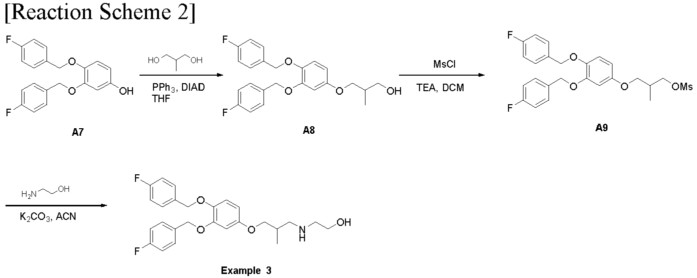

### Step 1) Synthesis of A8

A solution of A7 (3,4-bis((4-fluorobenzyl)oxy)phenol, 2 g, 5.84 mmol) in tetrahydrofuran (THF, 20 mL) were added 2-methylpropane-1,3-diol (0.63 g, 7.01 mmol), triphenylphospine (PPh₃, 2.29 g, 8.76 mmol) and diisopropyl azodicarboxylate (DIAD, 1.77 g, 8.76 mmol). The mixture was stirred at 65 °C for 16 hrs. Water was added, the solution was extracted with EA (20 mL × 3). The combined organic layers were washed with brine, dried over Na₂SO₄ and concentrated to afford A8 (3-(3,4-bis((4-fluorobenzyl)oxy)phenoxy)-2-methylpropan-1-ol, 1 g) as yellow oil.

LCMS; Mass Calcd.: 414; MS Found: 415 [MS+1]].

### Step 2) Synthesis of A9

To a solution of A8 (0.5 g, 1.2 mmol) in DCM (15 mL) was added TEA (0.37 g, 3.6 mmol). Methanesulfonyl chloride (MsCl, 0.21 g, 1.8 mmol) was added at 0 °C. The reaction was stirred at room temperature for 4 hrs. Water was added and extracted with DCM (5 mLx3). The organic layer was concentrated to give A9 (3-(3,4-bis((4-fluorobenzyl)oxy)phenoxy)-2-methylpropyl methanesulfonate , 0.6g) as yellow oil.

LCMS; Mass Calcd.: 492; MS Found: 492.9, 493.9 [MS+1]].

### Step 3) Synthesis of Example 3

To a solution of **A9** (0.6 g, 1.22 mmol) in Acetonitrile (ACN) (10 mL) were added 2-aminoethanol (74 mg, 1.22 mmol) and Potassium carbonate (K₂CO₃, 252 mg, 1.83 mmol). The mixture was stirred at 80 °C for 16 hrs. The reaction was was purified by Prep-HPLC to afford Example 3 (2-((3-(3,4-bis((4-fluorobenzyl)oxy)phenoxy)-2-methylpropyl)amino)ethan-1-ol, 18 mg) as a white solid.

¹H NMR (400 MHz, CD3OD): δ 8.55 (s, 1 H), 7.49-7.40 (m, 4 H), 7.13-7.03 (m, 4 H), 6.94 (d, 1 H), 6.74 (s, 1 H), 6.53-6.50 (m, 1 H), 5.09 (s, 1 H), 5.01 (s, 1 H), 4.02 (m, 1 H), 3.89-3.82 (m, 3 H), 3.21-3.08 (m, 4 H), 2.45 (m, 1 H), 1.14 (d/d, 1 H)

LCMS; Mass Calcd.: 457; MS Found: 457.9 and 459.0 [MS+2]].

### Example 4. Preparation of (S)-1-(3,4-bis((4-fluorobenzyl)oxy)phenoxy)-3-((2-hydroxyethyl)amino)propan-2-ol

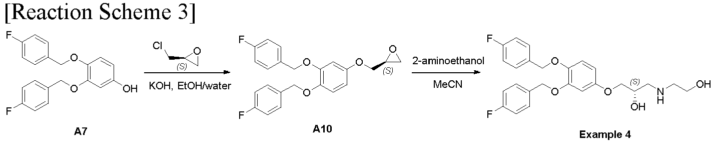

### Step 1) Synthesis of A10

To a solution of A7 (24.0 g, 70.2 mmol, 1.0 eq) in ethanol(EtOH, 240 mL) was added potassium hydroxide (KOH, 4.71 g, 84.2 mmol, 1.2 eq) and water (24 mL), then the solution was added (S)-2-(chloromethyl)oxirane (19.3 g, 210 mmol, 3.0 eq) at room temperature. The mixture was stirred at 30 °C for 16 hrs. The reaction solution was added water (240 mL) and filered, the solid was dried in vacuum to give **A10** ((*S*)-2-((3,4-bis((4-fluorobenzyl)oxy)phenoxy)methyl)oxirane, 21.0 g, yield 75.3%) as a yellow solid.

¹HNMR (DMSO-d₆, 400 MHz): δ7.43-7.51 (m, 4H), 7.16-7.25 (m, 4H), 6.94 (d, J = 8.8 Hz, 1H), 6.73 (d, J = 2.8 Hz, 1H), 6.44-6.47 (m, 1H), 5.10 (s, 2H), 5.00 (s, 2H), 4.22-4.26 (m, 1H), 3.73-3.77 (m, 1H), 3.29-3.30 (m, 1H), 2.83 (t, J = 4.8 Hz, 1H), 2.67-2.69 (m, 1H).

### Step 2) Synthesis of Example 4

A solution of **A10** ((*S*)-2-((3,4-bis((4-fluorobenzyl)oxy)phenoxy)methyl)oxirane , 25.0 g, 62.8 mmol, 1.0 eq) and 2-aminoethanol (19.5 g, 314 mmol, 5.0 eq) in acetonitrile (MeCN, 250 mL) was stirred at 50 °C for 16 hrs. The mixture was concentrated and The reaction mixture was pour into water (300 mL) and extracted with DCM (500 mL × 3). The organic layer was washed with brine (500 mL), dried over Na₂SO₄ and concentrated. The crude product was purified by flash column (DCM/MeOH= 5:1) to give **Example 4** ((*S*)-1-(3,4-bis((4-fluorobenzyl)oxy)phenoxy)-3-((2-hydroxyethyl)amino)propan-2-ol, 10.5 g, yield 34.5%) as a white solid. (TLC: DCM/MeOH=5/1, R_{f}=0.5)
¹HNMR (DMSO-d₆, 400 MHz): δ7.43-7.51 (m, 4H), 7.17-7.25 (m, 4H), 6.93 (d, J = 9.2 Hz, 1H), 6.68 (d, J = 2.4 Hz, 1H), 6.42-6.45 (m, 1H), 5.10 (s, 2H), 5.00 (s, 2H), 4.95 (s, 1H), 4.50 (s, 1H), 3.78-3.88 (m, 3H), 3.45 (d, J = 4.4 Hz, 2H), 2.67-2.68 (m, 1H), 2.54-2.65 (m, 3H), 1.91 (br s, 1H).

LCMS; Mass Calcd.:459; MS Found: 460.2 [MS+1].

### Example 5. Preparation of (R)-1-(4-((4-fluorobenzyl)oxy)phenoxy)-3-((2-hydroxyethyl)amino)propan-2-ol

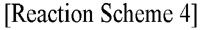

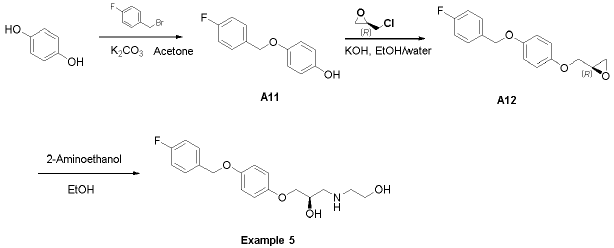

### Step 1) Synthesis of A11

To a solution of hydroquinone (0.88 g, 8.0 mmol) and K₂CO₃ (0.54 g, 4.0 mmol) in acetone (20 mL) was added 4-fluorobenzyl bromide (0.50 mL, 4.0 mmol). The mixture was refluxed for 6 hrs. After the reaction was completed, the resulting mixture was cooled and filtrate to remove salt. The residue was purified by flash column chromatography (n-hexane/ethyl acetate = 5/1) to give A11 (4-((4-fluorobenzyl)oxy)phenol, 0.30 g, 1.38 mmol, yield: 35%).

### Step 2) Synthesis of A12

To a solution of **A11** (4-((4-fluorobenzyl)oxy)phenol, 0.30 g, 1.38 mmol) in EtOH (13.8 mL) were added (R)-epichlorohydrine (0.54 ml, 6.90 mmol) and 2.5M aqueous KOH solution (1.1 mL, 2.76 mmol). The mixture was stirred at room temperature for 5 hrs. After reaction was completed, the mixture was evaporated to remove EtOH and partitioned between water (20 mL) and ethyl acetate (20 mL). The organic layer was extracted, dried over anhydrous magnesium sulfate (MgSO₄) and concentrated under reduced pressure. The residue was purified by flash column chromatography (n-hexane/ethyl acetate = 5/1) to give **A12** ((R)-2-((4-((4-fluorobenzyl)oxy)phenoxy)methyl)oxirane, 90 mg, 0.33 mmol, yield: 24%).

LCMS Calcd m/z for C₁₆H₁₅FO [M+H] ⁺ 275.11 Found 275.

### Step 3) Synthesis of Example 5

To a solution of **A12** (90 mg, 0.33 mmol) in EtOH (5 mL) was added 2-aminoethanol (0.10 mL, 1.64 mmol). The mixture was stirred at room temperature for 36 hrs. After reaction was completed, the mixture was evaporated to remove EtOH and partitioned between water (10 mL) and dichloromethane (10 mL). The organic layer was washed with brine, dried over anhydrous magnesium sulfate (MgSO₄) and concentrated under reduced pressure. The residue was purified by flash column chromatography (NH-silica; dichloromethane/MeOH = 20/1) to give **Example 5** ((R)-1-(4-((4-fluorobenzyl)oxy)phenoxy)-3-((2-hydroxyethyl)amino)propan-2-ol, 52mg, 0.16 mmol, yield: 48%) as a white solid.

¹H NMR (DMSO-d₆, 600 MHz) δ (ppm) 2.54-2.60 (m, 3H), 2.64-2.67 (m, 1H), 3.45 (brs, 2H), 3.78-3.87 (m, 3H), 4.49 (brs, 1H), 4.96 (brs, 1H), 5.01 (s, 2H), 6.86 (d, J = 9.0 Hz, 2H), 6.92 (d, J = 9.0 Hz, 2H), 7.21 (dd, J = 9.0 Hz, 9.0 Hz, 2H), 7.48 (dd, J=9.0 Hz, 5.4 Hz, 2H)
LCMS Calcd m/z for C₁₈H₂₂FNO₄ [M+H] ⁺ 336.16 Found 336.

### Example 6. Preparation of (R)-1-((2-aminoethyl)amino)-3-(3,4-bis((4-fluorobenzyl)oxy)phenoxy)propan-2-ol

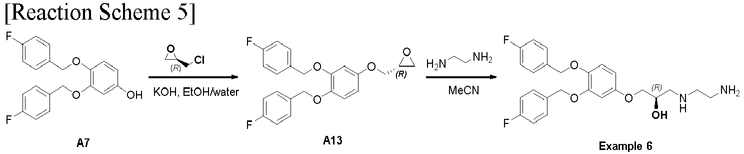

### Step 1) Synthesis of A13

To a solution of **A7** (3,4-bis((4-fluorobenzyl)oxy)phenol, 24.0 g, 70.2 mmol, 1.0 eq) in EtOH (240 mL) was added KOH (4.71 g, 84.2 mmol, 1.2 eq) and water (24 mL), then the solution was added (R)-2-(chloromethyl)oxirane (19.3 g, 210 mmol, 3.0 eq) at room temperature. The mixture was stirred at 30 °C for 16 hrs. The reaction solution was added water (240 mL) and file red, the solid was dried in vacuum to give **A13** ((*R*)-2-((3,4-bis((4-fluorobenzyl)oxy)phenoxy)methyl)oxirane, 19.0 g, yield 68.0%) as a yellow solid. (TLC: PE/EA=3/1, R_{f}=0.6).

¹H NMR (DMSO-d₆, 400 MHz): δ7.43-7.51 (m, 4H), 7.16-7.25 (m, 4H), 6.94 (d, J = 8.8 Hz, 1H), 6.73 (d, J = 2.8 Hz, 1H), 6.44-6.47 (m, 1H), 5.10 (s, 2H), 5.00 (s, 2H), 4.22-4.26 (m, 1H), 3.73-3.77 (m, 1H), 3.29-3.30 (m, 1H), 2.83 (t, J = 4.8 Hz, 1H), 2.67-2.69 (m, 1H).

### Step 2) Synthesis of Example 6

A solution of **A13** (24.0 g, 60.3 mmol, 1.0 eq) and ethane-1,2-diamine (18.1 g, 302 mmol, 5.0 eq) in MeCN (250 mL) was stirred at 50 °C for 16 hrs. The mixture was concentrated and the crude product was purified by prep-HPLC. The solution was concentrated to remove MeCN and the solution was adjusted pH=8 with aq.NaHCO₃ solution. The solid was filtered and washed with water (50 mLx3). The solid was dried in vacuum to give **Example 6** ((*R*)-1-((2-aminoethyl)amino)-3-(3,4-bis((4-fluorobenzyl)oxy)phenoxy)propan-2-ol, 10.0 g, yield 36.2%) as a white solid. (TLC: DCM/MeOH=5/1, R_{f}=0.2)

¹H NMR (CD₃OD, 400 MHz): δ7.40-7.49 (m, 4H), 7.03-7.12 (m, 4H), 6.93 (d, J = 8.4 Hz, 1H), 6.69 (d, J = 2.8 Hz, 1H), 6.47-6.50 (m, 1H), 5.08 (s, 2H), 5.00 (s, 2H), 4.02-4.04 (m, 1H), 3.89-3.92 (m, 2H), 2.70-2.84 (m, 6H).

¹H NMR (DMSO-d₆, 400 MHz): δ7.43-7.51 (m, 4H), 7.16-7.25 (m, 4H), 6.93 (d, J = 8.8 Hz, 1H), 6.69 (d, J = 2.4 Hz, 1H), 6.42-6.45 (m, 1H), 5.10 (s, 2H), 5.00 (s, 2H), 3.78-3.88 (m, 3H), 2.98-3.30 (br s, 2H), 2.50-2.66 (m, 6H).

LCMS:458; MS Found: 458.9 [MS+1].

### Example 7. Preparation of 3,4-bis(benzyloxy)-N-(2-hydroxyethyl)benzamide

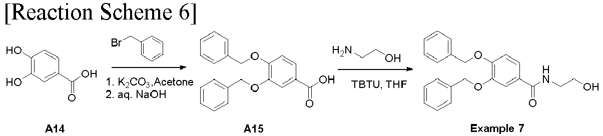

### Step 1) Synthesis of A15

To a solution of **A14** (3,4-dihydroxybenzoic acid, 200 mg, 1.29 mmol, 1.0 eq) in Acetone (6 mL) was added potassium carbonate (K₂CO₃, 717 mg, 5.18 mmol, 4.0 eq). Then the solution was added benzyl bromide (666 g, 3.89 mmol, 3.0 eq) at room temperature. The mixture was stirred at 70 °C for overnight. After cooling to room temperature, the reaction solution was diluted with EA, and washed with water. The organic layer was washed with brine, dried over anhydrous magnesium sulfate (MgSO₄) and concentrated under reduced pressure. The residue was purified by flash column chromatography (Hexane : EA = 10 : 1) to obtain benzyl 3,4-bis(benzyloxy)benzoate . The carbamate intermediate was diluted with MeOH, and aq. NaOH was added and heating to 60 °C for 3 hrs. After cooling to room temperature, the solution was adjusted pH=3 with aq.HCl solution. The solid was filtered and the filter cake was dried in vacuum to obtain **A15** (3,4-bis(benzyloxy)benzoic acid, 433 mg) as a white solid.

LCMS:334; MS Found: 334 [MS].

### Step 2) Synthesis of Example 7

To a mixture of **A15** (70 mg, 0.21 mmol, 1.0 eq), TBTU(CAS No. : 125700-67-6, 134 mg, 0.418 mmol) in anhydrous THF (4 mL), 2-aminoethan-1-ol (20 mg, 0.31 mmol, 1.5 eq) and Et₃N(64 mg, 0.627 mmol) was added and stirred at room temperature for 16 hrs. The reaction solution was diluted with EA, and washed with water. The organic layer was washed with brine, dried over anhydrous magnesium sulfate (MgSO₄) and concentrated under reduced pressure. The residue was purified by flash column chromatography (DCM : MeOH = 95 : 5) to obtain Example 7 (3,4-bis(benzyloxy)-N-(2-hydroxyethyl)benzamide, 45 mg, 57% yield) as a white solid.

¹H NMR (DMSO-d₆, 400 MHz): δ 8.33 (s, 1H), 7.59 (s, 1H), 7.47-7.44 (m, 4H), 7.40-7.38 (m, 4H), 7.37 (m, 2H), 7.12 (s, 1H), 5.19 (s, 2H), 5.16 (s, 2H), 4.74 (t, 1H), 3.48 (m, 2H), 3.30 (m, 2H)
LCMS:377; MS Found: 378 [MS+1].

### Example 8. Preparation of (R)-1-((4-((4-fluorobenzyl)oxy)phenyl)(methyl)amino)-3-((2-hydroxyethyl)amino)propan-2-ol

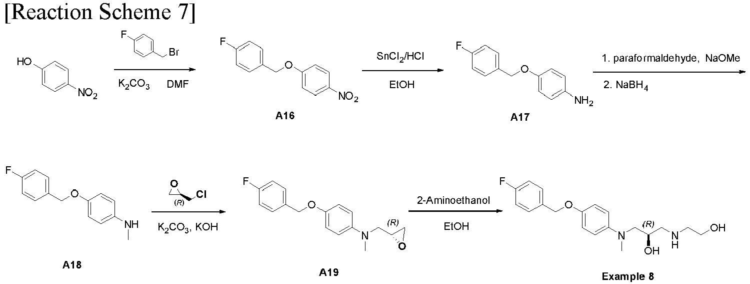

### Step 1) Synthesis of A16

To a solution of 4-nitrophenol (0.42g, 3.0 mmol) in anhydrous DMF (3 mL) were added 4-fluorobenzyl bromide (0.37 mL, 3.0 mmol) and K₂CO₃ (0.83 g, 6.0 mmol). The mixture was stirred at 90 °C for 3 hrs. After the reaction was completed, the mixture was cooled and poured into H₂O (20 mL). The precipitated solid was collected and dissolved in ethyl acetate (20 mL) The residue was washed with brine, dried over anhydrous MgSO₄ and concentrated in vacuo to give A16 (1-fluoro-4-((4-nitrophenoxy)methyl)benzene, 0.56g, 2.27 mmol, yield: 76%).

### Step 2) Synthesis of A17

To a solution of **A16** (0.56g, 2.27 mmol) in EtOH (10 mL) were added SnCl₂ (0.97g, 5.11 mmol) and conc. HCl (2 mL). The mixture was stirred at 60 °C for 4 hrs. After the reaction was completed, the mixture was evaporated to remove EtOH and partitioned between saturated NaHCO₃ aqueous solution (50 mL) and ethyl acetate (50 mL). The organic layer was washed with brine, dried over anhydrous MgSO₄ and concentrated under reduced pressure. The residue was purified by flash column chromatography (n-hexane/ethyl acetate = 5/1~1/1) to give **A17** (4-((4-fluorobenzyl)oxy)aniline, 0.18g, 0.83 mmol, yield: 37%)

LCMS Calcd m/z for C₁₃H₁₂FNO [M+H]⁺ 218.10 Found 218.

### Step 3) Synthesis of A18

To a solution of **A17** (0.18g, 0.83 mmol) and paraformaldehyde (99 mg, 3.31 mmol) in MeOH (5 mL) was added 5.0 M NaOMe in MeOH (0.33 mL, 1.65 mmol) at 0 °C by dropwise manner. The mixture was refluxed for 1 hr. After the imine formation was completed, NaBH₄ (125mg, 3.31 mmol) was added to the mixture at 0 °C. Then, the mixture was refluxed for another 1 hr. The resulting mixture was evaporated to remove MeOH and partitioned between water (10 mL) and dichloromethane (10 mL). The organic layer was washed with 1 N NaOH aqueous solution, dried over anhydrous MgSO₄ and concentrated under reduced pressure. The residue was purified by flash column chromatography (n-hexane/ethyl acetate = 3/1) to give **A18** (4-((4-fluorobenzyl)oxy)-N-methylaniline, 50 mg, 0.22 mmol, yield: 27%)
LCMS: Calcd m/z for C₁₄H₁₄FNO [M+H]⁺ 232.12 Found 232.

### Step 4) Synthesis of A19

To a solution of A18 (50 mg, 0.22 mmol) in (S)-epichlorohydrine (0.17 mL, 2.2 mmol) were added K₂CO₃ (45 mg, 0.33 mmol) and crushed KOH (123 mg, 2.2 mmol, portion drop) at 0 °C. The mixture was stirred at room temperature for overnight. The resulting mixture was partitioned between water (10 mL) and ethyl acetate (10 mL). The organic layer was washed with brine, dried over anhydrous MgSO₄ and concentrated under reduced pressure. The residue was purified by flash column chromatography (n-hexane/ethyl acetate = 5/1~2/1) to give **A19** ((*R*)-4-((4-fluorobenzyl)oxy)-N-methyl-N-(oxiran-2-ylmethyl)aniline, 30 mg, 0.10 mmol, yield: 45%).

LCMS: MS Calcd m/z for C₁₇H₁₈FNO₂ [M+H]⁺ 288.14 Found 288.

### Step 5) Synthesis of Example 8

To a solution of **A19** (30 mg, 0.10 mmol) in EtOH (5 mL) was added 2-aminoethanol (0.03 mL, 0.50 mmol). The mixture was stirred at room temperature for overnight. After reaction was completed, the mixture was evaporated to remove EtOH and partitioned between water (10 mL) and dichloromethane (10 mL). The organic layer was washed with brine, dried over anhydrous MgSO₄ and concentrated under reduced pressure. The residue was purified by flash column chromatography (NH-silica; dichloromethane/MeOH = 100/1~20/1) To give **Example 8** ((*R*)-1-((4-((4-fluorobenzyl)oxy)phenyl)(methyl)amino)-3-((2-hydroxyethyl)amino)propan-2-ol, 8mg, 0.02 mmol, yield: 20%) as a white solid.

¹H NMR (DMSO-d₆, 600 MHz) δ (ppm) 2.43-2.46 (m, 1H), 2.52-2.58 (m, 3H), 2.85 (s, 3H), 3.08-3.12 (m, 1H), 3.27-3.31 (m, 1H), 3.42-3.46 (m, 2H), 3.71-3.76 (m, 1H), 4.49 (brs, 1H), 4.74 (brs, 1H), 4.96 (s, 2H), 6.64 (d, J = 9.0 Hz, 2H), 6.84 (d, J = 9.0 Hz, 2H), 7.20 (dd, J = 9.0 Hz, 9.0 Hz, 2H), 7.46 (dd, J = 9.0 Hz, 5.4 Hz, 2H)
LCMS: MS Calcd m/z for C₁₉H₂₅FN₂O₃ [M+H]⁺ 349.19 Found 349.

### Example 9. Preparation of (S)-1-(3-((4-fluorobenzyl)oxy)phenoxy)-3-((2-hydroxyethyl)amino)propan-2-ol

Example 9 was synthesized as white solid ((*S*)-1-(3-((4-fluorobenzyl)oxy)phenoxy)-3-((2-hydroxyethyl)amino)propan-2-ol, 34.8 % yield) in the similar manner as in Example 4 by using 3-((4-fluorobenzyl)oxy)phenol as a starting material instead of A7 (3,4-bis((4-fluorobenzyl)oxy)phenol).

¹H NMR (DMSO-d₆, 400 MHz): δ 7.53 (m, 2H), 7.23 (m 2H), 7.20 (m, 1H), 6.62-6.54 (m, 3H), 5.14 (m, 2H), 3.85 (m, 1H), 3.78 (m, 2H), 3.48 (m, 2H), 2.66-2.58 (m, 4H).

### Example 10. Preparation of (R)-1-(3-chloro-5-((4-fluorobenzyl)oxy)phenoxy)-3-((2-hydroxyethyl)amino)propan-2-ol

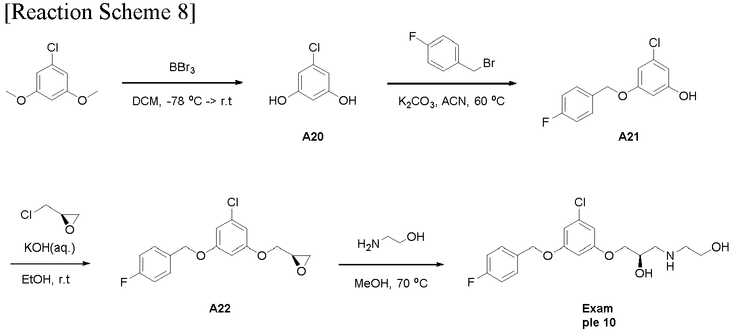

### Step 1) Synthesis of A20

To a solution of 1-chloro-3,5-dimethoxybenzene (1.85 g, 10.7 mmol) in DCM (100 mL) was added BBr₃ (1M in DCM, 32 mL, 32.2 mmol) at. -78 °C slowly. The reaction mixture was stirred for overnight at room temperature. The mixture was quenched with water and extracted with DCM, NaHCO₃ and brine. The organic layer was dried over anhydrous MgSO₄, filtered it and evaporated in vacuo. The residue was purified by column chromatography (EA/Hex = 1/4~2/5) to afford **A20** (5-chlorobenzene-1,3-diol, 1.54 g, 10.7 mmol, yield : 100%) as brown oil.

LCMS: MS Calcd m/z for C₆H₅ClO₂ [M+H]⁺ 144.99 Found 145.10

### Step 2) Synthesis of A21

To a solution of **A20** (1.54 g, 10.7 mmol) in ACN (20 mL) were added 1-(bromomethyl)-4-fluorobenzene (1.3 mL, 10.7 mmol) and K₂CO₃ (2.22 g, 16.1 mmol) at room temperature. The reaction mixture was stirred at 60 °C for overnight. The reaction mixture was extracted with EA and water. The organic layer was dried over anhydrous MgSO₄, filtered it and evaporated in vacuo. The residue was purified by column chromatography (EA/Hex = 1/9~3/7) to afford **A21** (3-chloro-5-((4-fluorobenzyl)oxy)phenol, 748.2 mg, 2.96 mmol, yield : 27 %) as yellow oil.

¹H NMR (DMSO-d₆, 600 MHz) δ (ppm) 9.93 (brs, 1H), 7.47 (dd, J = 10.4, 3.8 Hz, 2H), 7.24-7.15 (m, 2H), 6.52 (t, J = 2.0 Hz, 1H), 6.41 (t, J = 1.9 Hz, 1H), 6.35 (t, J = 2.1 Hz, 1H), 5.04 (s, 2H)
LCMS: MS Calcd m/z for C₁₃H₁₀ClFO₂ [M+H]⁺ 253.04 Found 253.10

### Step 3) Synthesis of A22

To a solution of **A21** (748.2 mg, 2.96 mmol) in EtOH (30 mL) was added (*R*)-2-(chloromethyl)oxirane (1.16 mL, 14.8 mmol). Then KOH (332.2 mg, 5.92 mmol) in H₂O (1mL) was added to the reaction mixture. The mixture was stirred at room temperature for 6 hrs and evaporated in vacuo. The residue was diluted with EA and extracted with EA and H₂O. The organic layer was dried over anhydrous MgSO₄, filtered it and evaporated in vacuo. The residue was purified by column chromatography (EA/Hex = 1/4~2/5) to afford **A22** ((*R*)-2-((3-chloro-5-((4-fluorobenzyl)oxy)phenoxy)methyl)oxirane, 313.1 mg, 1.01 mmol, yield : 35 %) as yellow oil.

LCMS: MS Calcd m/z for C₁₆H₁₄ClFO₃ [M+H]⁺ 309.06 Found 309.50

### Step 4) Synthesis of Example 10

To a solution of **A22** (313.1 mg, 1.01 mmol) in MeOH (3 mL) was added 2-aminoethanol (0.18 mL, 3.04 mmol). The reaction mixture was refluxed for overnight at 70°C. Then the mixture was evaporated in vacuo and purified by column chromatography (DCM/MeOH= 95/5~85/15) to afford **Example 10** ((*R*)-1-(3-chloro-5-((4-fluorobenzyl)oxy)phenoxy)-3-((2-hydroxyethyl)amino)propan-2-ol, 159.7 mg, 0.43 mmol, yield : 43 %).

¹H NMR (DMSO-d₆, 600 MHz) δ (ppm) 7.49 (dd, J = 8.4, 5.7 Hz, 2H), 7.22 (t, J = 8.8 Hz, 2H), 6.68 (t, J = 1.8 Hz, 1H), 6.62 (t, J = 1.8 Hz, 1H), 6.56 (t, J = 2.0 Hz, 1H), 5.09 (s, 2H), 4.99 (brs, 1H), 4.48 (s, 1H), 3.95 (dd, J = 9.6, 4.1 Hz, 1H), 3.87-3.83 (m, 2H), 3.44 (s, 2H), 2.65-2.53 (m, 4H)
LCMS: MS Calcd m/z for C₁₈H₂₁ClFNO₄ [M+H]⁺ 370.11 Found 370.20.

### Example 11. Preparation of 2-((3-(3,4-bis((4-fluorobenzyl)oxy)phenoxy)propyl)amino)ethan-1-ol

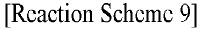

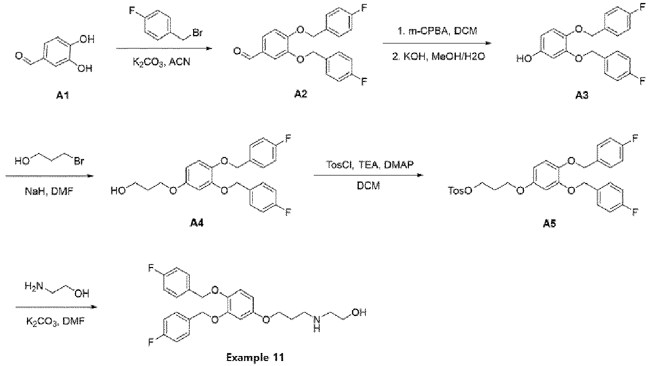

### Step 1) Synthesis of A2

To a solution of **A1** (100 g, 724 mmol, 1.0 eq) inACN (2000 mL) were added K₂CO₃ (500 g, 3.62 mol, 5.0 eq) and 1-(bromomethyl)-4-fluorobenzene (342 g, 1.81 mol, 2.5 eq). The mixture was refluxed overnight. The reaction was filtered and concentrated. The residue was added PE/EA (20/1, 1000 mL) and stirred for 1 hr, then filtered to afford **A2** (3,4-bis((4-fluorobenzyl)oxy)benzaldehyde, 150 g, 58.5%) as a white solid. (TLC: PE/EA=5/1, R_{f}=0.5).

¹HNMR (CDCl₃, 400 MHz): δ 9.85 (s, 1H), 7.51-7.41 (m, 6H), 7.11-7.03 (m, 5H), 5.21 (s, 2H), 5.17 (s, 2H)
LCMS; Mass Calcd.:354.10; MS Found: 355.10 [MS+1].

### Step 2) Synthesis of A3

To a solution of **A2** (150 g, 423 mmol, 1.0 eq) in DCM (800 mL) was added 3-chloroperbenzoic acid (110 g, 635 mmol, 1.5 eq) at room temperature. The mixture was stirred for 1 hr at room temperature. Then the reaction was quenched by NaHCO₃ solution, extracted with DCM, and concentrated. The residue in MeOH (300 mL) was added 5N KOH solution (93.1 mL, 466 mmol, 1.1 eq) and stirred for 1 hr at room temperature. The pH was adjusted to 6 with 1N HCl solution. Then added water and filtered to afford A3 (3,4-bis((4-fluorobenzyl)oxy)phenol, 100 g, crude, 69.0 %) as a white solid. (TLC: PE/EA=3/1, R_{f}=0.5)

¹HNMR (CDCl₃, 400 MHz): δ 7.42-7.36 (m, 4H), 7.09-7.01 (m, 4H), 6.80 (d, J = 8.4 Hz, 1H), 6.50 (d, J = 2.8 Hz, 1H), 6.33 (dd, J = 8.4, 2.8 Hz, 1H), 5.06 (s, 2H), 5.01 (s, 2H).

LCMS; Mass Calcd.:342.10; MS Found: 343.10 [MS+1].

### Step 3) Synthesis of A4

To a solution ofNaH (12.9 g, 60% dispersed in mineral oil, 321 mmol, 1.1 eq) was added a solution of A3 (100 g, 292 mmol, 1.0 eq) in DMF (1000 mL) under N₂ at 0°C. The mixture was stirred for 30 min at room temperature and a solution of 3-bromo-1-propanol (44.3 g, 321 mmol, 1.1 eq) in DMF (200 mL) was added at this temperature. The resulting mixture was stirred for 2 hrs at room temperature. The reaction was quenched by the addition of saturated aqueous NH₄Cl. Then extracted with EA (600 mL). The combined organic layer was dried over anhydrous Na₂SO₄, filtrated, concentrated under vacuum. The residue was purified by chromatography on silica gel with (PE/EA =30/1-3/1) to afford **A4** (3-(3,4-bis((4-fluorobenzyl)oxy)phenoxy)propan-1-ol, 80.0 g, 68.4%) as a white solid. (TLC: PE/EA=3/1, R_{f}=0.5)

¹HNMR (CDCl₃, 400 MHz): δ 7.43-7.36 (m, 4H), 7.09-7.01 (m, 4H), 6.87 (d, J = 8.8 Hz, 1H), 6.58 (d, J = 2.8 Hz, 1H), 6.43 (dd, J = 8.8, 2.8 Hz, 1H), 5.08 (s, 2H), 5.02 (s, 2H), 4.06 (t, J = 6 Hz, 2H), 3.87 (t, J = 6 Hz, 2H), 2.04-2.01 (m, 2H).

LCMS; Mass Calcd.:400.15; MS Found: 401.10 [MS+1].

### Step 4) Synthesis of A5

To a solution of **A4** (80.0 g, 200 mmol, 1.0 eq) in DCM (800 mL) were added TEA (40.4 g, 400 mmol, 2.0 eq), 4-dimethylaminopyridine (2.44 g, 20.0 mmol, 0.1 eq), and p-toluenesulfonyl chloride (76.3 g, 400 mmol, 2.0 eq). The mixture was stirred at room temperature overnight. TLC showed the reaction was the complete. The reaction was quenched by water (1000 mL) and extracted with DCM (500 mL). The organic layers were combined and washed with brine. The residue was applied onto a silica gel column (PE/EA =20/1-5/1) to afford **A5** (3-(3,4-bis((4-fluorobenzyl)oxy)phenoxy)propyl 4-methylbenzenesulfonate, 70 g, 63.1 %) as a white solid. (TLC: PE/EA=3/1, R_{f}=0.8)

¹HNMR (CDCl₃, 400 MHz): δ 7.77 (d, J = 8.4 Hz, 2H), 7.44-7.37 (m, 4H), 7.29-7.27 (m, 2H), 7.10-7.02 (m, 4H), 6.84 (d, J = 8.8 Hz, 1H), 6.48 (d, J = 2.8 Hz, 1H), 6.30 (dd, J = 11.6, 3.2 Hz, 1H), 5.07 (s, 2H), 5.03 (s, 2H), 4.24 (t, J = 6 Hz, 2H), 3.90 (t, J = 6 Hz, 2H), 2.41 (s, 3H), 2.11-2.08 (m, 2H).

LCMS; Mass Calcd.:554.16; MS Found: 555.10 [MS+1].

### Step 5) Synthesis of 2-((3-(3,4-bis((4-fluorobenzyl)oxy)phenoxy)propyl)amino)ethan-1-ol

To a solution of **A5** (70 g, 126 mmol, 1.0 eq) in DMF (700 mL). Then added K₂CO₃ (52.3 g, 379 mmol, 3.0 eq), 2-aminoethanol (38.5 g, 631 mmol, 5.0 eq). Then the mixture was stirred at 80 °C overnight. The reaction was quenched by cold water (1000 mL) and extracted with EA (700 mL). The combined organic layer was dried over anhydrous Na₂SO₄, concentrated under vacuum. Another 35 g batch was carried out as the above procedure. The residue was purified by chromatography on silica gel with (DCM/MeOH =50/1-10/1) to afford Example 11 (2-((3-(3,4-bis((4-fluorobenzyl)oxy)phenoxy)propyl)amino)ethan-1-ol, 30 g, 53.7 %) as a white solid. (TLC: DCM/MeOH=10/1, R_{f}=0.4).

¹HNMR (DMSO-d₆, 400 MHz): δ 7.51-7.42 (m, 4H), 7.25-7.16 (m, 4H), 6.93 (d, J = 8.8 Hz, 1H), 6.68 (d, J = 2.4 Hz, 1H), 6.42 (dd, J = 8.8, 2.4 Hz, 1H), 5.09 (s, 2H), 4.99 (s, 2H), 3.94 (t, J = 6 Hz, 2H), 3.48 (t, J = 5.6Hz, 2H), 2.69 (t, J = 7.2 Hz, 2H), 2.63 (t, J = 5.6 Hz, 2H), 1.85-1.82 (m, 2H) ; 13CNMR (DMSO-d6, 100 MHz): δ 163.41, 163.35, 160.99, 160.93, 154.23, 149.83, 142.51, 134.31, 134.28, 133.90, 133.87, 13026, 13024, 130.18, 130.15, 116.99, 115.79, 115.58, 115.44, 115.60, 103.31, 71.04, 69.76, 66.56, 60.35, 51.92, 46.31, 29.37
LCMS; Mass Calcd.:443.19; MS Found: 444.90 [MS+1].

### [Experimental Example 1] Confirmation of the effect of bacterial infection on p62 and LC3 expression levels

The following experiment was performed to determine what effect bacterial infection had on the expression levels of autophagy-related genes such as p62 and LC3.

### [Experimental Example 1-1] Confirmation of inhibition of p62 and LC3 expression levels

First, J774A.1 macrophages (Korean Cell Line Bank) were cultured in RPMI (Rosewell Park Memorial Institue) supplemented with 10% FBS and 100 units/mL penicillin/streptomycin, and HeLa cells were cultured in Dulbecco's Modified Eagle's Medium (DMEM; Gibco, 10566016) supplemented with 10% FBS and 100 units/mL penicillin/streptomycin. All cell lines were tested negative for mycoplasma using the MycoAlert detection kit (Lonza, LT07-118), and all culture plates and cell lines were maintained in a humidified incubator at 37 °C and 5% COz.

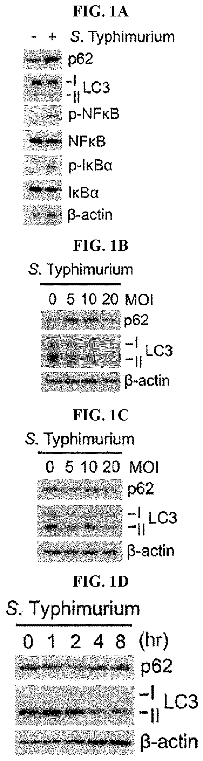

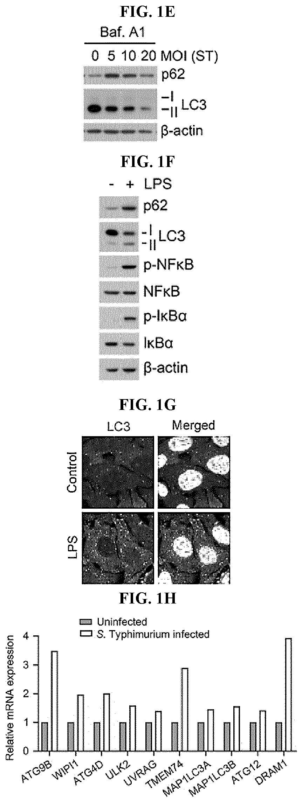

Macrophage J774A.1 and HeLa cells were infected with *Salmonella Typhimurium,* a Gram-negative bacterium, and the expression of LC3 was monitored through immunoblotting. Mouse monoclonal anti-p62 (Abeam, ab56416, 1: 100,000) was used for immunoblotting, rabbit polyclonal anti-LC3 (Sigma, L7543, 1:40,000) was used for immunoblotting, and anti-rabbit IgG-HRP (Cell Signaling, 7074, 1:10,000) and anti-mouse IgG-HRP (Cell Signaling, 7076, 1:1000) were used as secondary antibodies. FIG. 1A shows that Immunoblotting results are shown during S. Typhimurium infection (MOI 10).

As a result of comparing the expression level according to multiplicity of infection (MOI), as shown in Figs. 1B (J774A.1 cells) and 1C (HeLa cells), the expression level of p62 increased due to S. Typhimurium infection, whereas the expression level of LC3 decreased and lipidation into its activated form, LC3-II, decreased. In particular, it was confirmed that the expression levels of LC3-I and LC3-II decreased as the multiplicity of infection increased.

### [Experimental Example 1-2] Confirmation of effects of lysosomal activity inhibitor and LPS treatment

The J774A.1 cells of Experimental Example 1-1 were treated with bafilomycin A1 (100 nM), an inhibitor of lysosomal V-ATPase, for 4 hours, and immunoblotting was performed in the same manner as in Experimental Example 1-1. However, there was no difference in the degree of inhibition of LC3-I and LC-II expression even when treated with lysosomal V-ATPase inhibitor (FIG. 1E).

In addition, in order to rule out the possibility that J774A.1 and HeLa cells lack the ability to induce autophagy in response to pathogen invasion, the J774A.1 and HeLa cells of Experimental Example 1-1 were treated with lipopolysaccharide (LPS) (100 ng/mL), a component of the outer membrane of Gram-negative bacteria, as a pathogen-associated molecular pattern (PAMP) for 6 hours, and immunoblotting was performed in the same manner as in Experimental Example 1-1. As a result, it was confirmed that, in contrast to live bacteria, LPS strongly induced not only the synthesis of LC3 (LC3-I) but also its lipidation (LC3-II) (FIG. 1F).

### [Experimental Example 1-3] Confirmation of effect on expression of ATG9B, WIPI1, TMEM74, ULK2 and DRAM1

As an innate immune system that responds to S. Typhimurium (MOI 10), the HeLa cells of Experimental Example 1-1 were infected with S. Typhimurium (MOI 10). Then, mRNA expression levels of ATG9B, WIPI1, TMEM74, ULK2, and DRAM1, known as autophagy-related genes, were measured through quantitative real-time PCR (RT-qPCR).

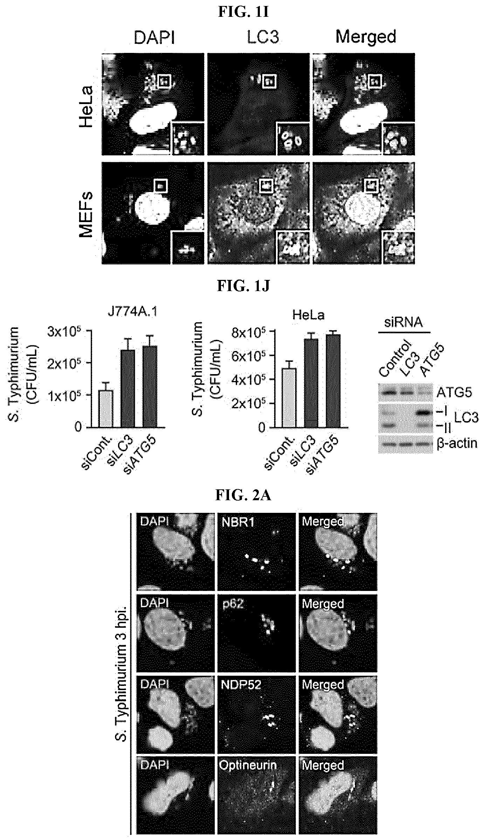

As a result, the expression levels of the above five genes increased (FIG. 1H), and approximately 30% of bacteria in both HeLa cells and MEF cells were found to be associated with LC3-positive autophagy membranes (FIG. 1I).

### [Experimental Example 1-4] Confirmation of changes in bacterial infectivity when gene expression of ATG5 and LC3B is suppressed

Changes in infectivity of S. Typhimurium were measured when gene expression of ATG5 and LC3B was suppressed using siRNA.

Specifically, J774A.1 and HeLa cells were transfected with ATG5 siRNA and LC3B siRNA (Map11c3b siRNA) (Life Technologies) using Lipofectamine RNAiMAX reagent (Invitrogen, 13778150) (#4392420, ID #23628) at a final concentration of 40 nM according to the manufacturing protocol.
ATG5 siRNA: 5'-CAGUAUCAGACACGAUCAU-3' (sense)(SEQ ID NO: 1) and 5'-AUGAUCGUGUCUGAUCUG-3' (antisense)(SEQ ID NO: 2)
LC3B siRNA: 5'-CCAAGAUCCAGUGAUUAUUUU-3'(sense)(SEQ ID NO: 3) and 5'-AUAAUCACUGGAUCUUGGUUCU-3' (antisense)(SEQ ID NO: 4)

Approximately 48 hours after siRNA silencing, cells were harvested for immunoblotting and immunocytochemical analysis, and the degree of colony formation was measured as follows. Single colonies of *S*. Typhimurium were grown overnight at 37°C in a shaking incubator, secondary cultures (5% inoculum) were grown for 3 hours under microaerobic conditions, and HeLa or J774A.1 cell lines were infected at an MOI of 10 for 30 minutes. Infected cells were washed with Dulbecco's Phosphate Buffered Salin (DPBS; Thermo Fisher Scientific, 14040133), and cells were treated with medium containing 100 µg/ml gentamycin (Thermo Fisher Scientific, 15710064) for 1 hour to measure extracellular Bacteria. The medium was replaced with fresh medium containing 10 µg/ml gentamicin. The cells were then lysed using lysis buffer (0.1% Triton X-100, 1X phosphate buffered saline (PBS; Welgene, LB 204-01)). Intracellular S. Typhimurium-containing lysates were serially diluted and spread on Luria broth (LB) agar plates. After overnight incubation at 37°C, CFU was measured.

As a result, when ATG5 and LC3B genes related to autophagy activity were deleted using siRNA, the infectivity of *S*. Typhimurium was promoted in J774A.1 and HeLa cells (FIG. 1J).

This showed that host cells infected with S. Typhimurium maintain significant autophagic activity.

### [Experimental Example 2] Confirmation of relationship between p62 protein expression and bacterial infectivity

Through Experimental Example 1, it was confirmed that the expression level of LC3 was reduced during bacterial infection, but the host cell itself maintained autophagic activity. Therefore, to confirm whether the expression of p62 protein is related to bacterial infectivity, immunocytochemistry was performed as follows.

Specifically, an autoclaved 22 mm² coverslip was placed in a 24-well plate, and HeLa cells of the same type as in Experimental Example 1 were seeded at 2.5 × 10 cells/well and cultured overnight. The cells were infected with S. Typhimurium S14028 at an MOI of 10, and the cells were fixed in 4% PFA in PBS for 15 minutes at room temperature. After washing twice with PBS, the cells were incubated in blocking solution (5% bovine serum albumin and 0.3% Triton X-100 in PBS) for 1 hour. The sample was incubated with the same type of primary antibody as in Experimental Example 1 at 4°C overnight, and the secondary antibody was incubated at room temperature. After washing the secondary antibody with PBS, the samples were mounted on coverslide with mounting solution. Confocal images were taken on a Zeeiss LSM 700 laser scanning confocal microscope equipped with Zeiss C-Apochromat 60x (1.2 NA) and 40x (l.2NA) water immersion lenses, and analyzed using ZEN (black edition) 2012 SP5 software (Zeiss). Using ZEN software, z-stacks of images covering the entire cell thickness were acquired and maximally projected. Image processing and annotation were performed using Adobe Photoshop, Adobe Illustor, and Fiji software, and the results are shown in FIGs. 2A to 2D.

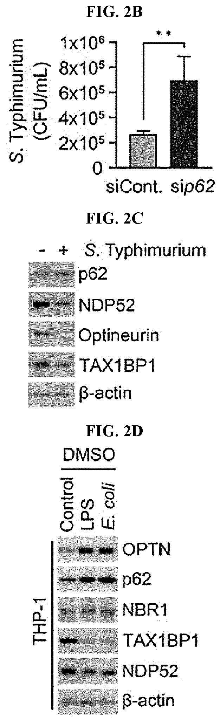

As shown in FIG. 2A, p62, NBR1, Optinurin, and NDP52 were found to be associated with bacterial membranes to varying degrees and with distinct spatiotemporal patterns (FIGs. 2A and 2C). In addition, when suppressing the expression of p62 using siRNA in the same manner as in Experimental Example 1-4, among the p62, NBR1, Optinurin, TAX1BP1, and NDP52 genes, it was confirmed that knockdown of p62 significantly affected the infectivity of *S*. Typhimurium (FIGs. 2B). Consistently, levels of p62 were reliably increased upon bacterial infection compared to TAX1BP1, NDP52, and Optinurin (FIGs. 2C and 2D).

Through this, it was found that p62 is a potential target for antibacterial substances.

### [Experimental Example 3] Confirmation of antibacterial efficacy

To confirm the antibacterial efficacy of the compounds of Examples 1 to 10 prepared in the above Preparation Example, the following colony formation measurement experiment was performed.

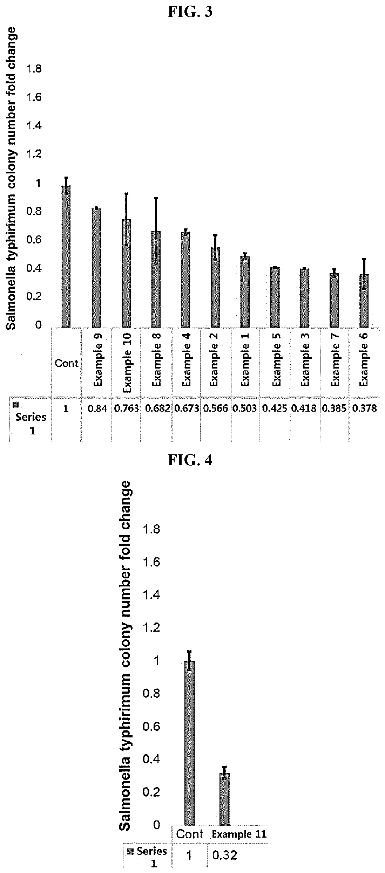

Raw264.7 macrophage cell line was cultured using DMEM medium containing 10% FBS and 1% streptomycin/penicillin in an incubator maintained at 5% carbon dioxide. To measure the bacteriophage efficacy according to treatment with the compounds of Examples 1 to 10, cells were seeded in a 12-well plate. An additional 24 hours of culture was performed to ensure that the cells were completely attached to the surface of the plate. Thereafter, 5×10⁴ cell lines were infected with *Salmonella Typhimurium* at an MOI of 10 for 30 minutes, and then treated with 2-10 uM of each of the compounds of Examples 1 to 11 for 5 hours, and then the cells were collected. To extract *Salmonella Typhimurium* that grew intracellularly from the collected cells, each sample was treated with 0.1%triton-x-100/PBS solution, and the extracted bacteria were cultured in LB agar colonies for 16 hours. The number of colonies produced after culturing was measured, and the results of the experiment are shown in FIGs. 3 and 4.

As shown in FIGs. 3 and 4, when treated with the compounds of Examples 1 to 11, the number of colonies produced by *Salmonella Typhimurium* propagated in the Raw264.7 cell line decreases compared to the control group, especially when treated with the compounds of Examples 1, 3, and 5 to 7, it was confirmed that the level was reduced to about 50% or less of the control group.

Through this, it was found that the compound according to one aspect of the present invention exhibits bacteriophage efficacy and has excellent antibacterial effect.

## Claims

1. A compound represented by the following Chemical Formula 1, a salt, a stereoisomer, a solvate or a hydrate thereof: 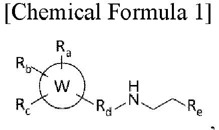 wherein in Chemical Formula 1,
W is phenyl;
Rₐ, R_{b} and R_{c} are each independently hydrogen, halogen, 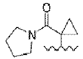 or -O-(CH₂)ₙ₁-R₁, R₁ is unsubstituted phenyl or halogen-substituted phenyl, and n1 is an integer from 1 to 3;
R_{d} is -CH₂-, -O-CH₂-CH(CH₃)-CH₂-, -O-(CH₂)ₙ₂-, -O-CH₂-CH(OH)-CH₂-, -(C=O)- or - N(CH₃)-CH₂-CH(OH)-CH₂-, and n2 is an integer of 1 to 5; and
Rₑ is -OH or -NH₂.

2. A composition for antibacterial use comprising the compound according to claim 1, a salt, a stereoisomer, a solvate or a hydrate thereof as an active ingredient.

3. A composition for inhibiting bacterial infectivity, comprising the compound according to claim 1, a salt, a stereoisomer, a solvate or a hydrate thereof as an active ingredient.

4. A composition for preventing, alleviating or treating bacterial infectious diseases, comprising the compound according to claim 1, a salt, a stereoisomer, a solvate or a hydrate thereof as an active ingredient.

5. A composition for preventing, alleviating or treating inflammation caused by bacterial infection, comprising the compound according to claim 1, a salt, a stereoisomer, a solvate or a hydrate thereof as an active ingredient.

6. The composition according to any one of claims 2 to 5, wherein the composition has antibacterial activity against Gram-negative or Gram-positive bacteria.

7. The composition according to claim 6, wherein the Gram-negative bacteria is one or more selected from the group consisting of *Salmonella* species, *Yersinia* species, *Escherichia* species, *Chlamydia* species, *Xanthomonas* species, *Erwinia* species, *Pseudomonas* species and *Ralstonia* species.

8. The composition according to claim 6, wherein the Gram-positive bacteria is one or more selected from the group consisting of *Mycobacterium* species, *Streptococcus* species, *Staphylococcus* species, *Enterococcus* species and *Lactobacillus* species.

9. The composition according to any one of claims 2 to 5, wherein the composition has antibacterial activity against bacteria resistant to antibiotics.

10. The composition according to claim 4, wherein the bacterial infectious diseases is one or more selected from the group consisting of pneumonia, bromatotoxism, dysentery, *Pseudomonas aeruginosa* infection, impetigo, purulent disease, acute dermatitis, cystitis, vaginitis, meningitis, typhoid, paratyphoid fever, tetanus, Lyme disease, Rocky Mountain spotted fever, cholera, leprosy, brucellosis, ehrlichiosis, Q fever, scarlet fever, listeriosis, botulism, leptospirosis, pestis, typhus, bacteremia, endocarditis, and enteritis.

11. The composition according to any one of claims 2 to 5, wherein the composition is a pharmaceutical, food or cosmetic composition.
